(19) Europäisches Patentamt
European Patent Office
Office européen des brevets

(11) Publication number: 0 196 024
B1

(12) **EUROPEAN PATENT SPECIFICATION**

(45) Date of publication of patent specification: 22.11.90

(21) Application number: 86103874.3

(22) Date of filing: 21.03.86

(51) Int. Cl.[5]: C 07 D 491/147, A 61 K 31/435, A 61 K 31/495 // (C07D491/147, 307:00, 221:00, 221:00),(C07D491/147, 311:00, 221:00, 221:00),(C07D491/147, 311:00, 241:00, 221:00)

(54) **Substituted 1,8-naphthyridinones, processes for their preparation and pharmaceutical compositions containing them.**

(30) Priority: 25.03.85 US 716003
10.03.86 US 836357

(43) Date of publication of application:
01.10.86 Bulletin 86/40

(45) Publication of the grant of the patent:
22.11.90 Bulletin 90/47

(84) Designated Contracting States:
AT BE CH DE FR GB IT LI LU NL SE

(56) References cited:
EP-A-0 092 786
EP-A-0 127 135

The Merck Manual (1982) 14th edition, p. 265

The file contains technical information submitted after the application was filed and not included in this specification

(73) Proprietor: SCHERING CORPORATION
2000 Galloping Hill Road
Kenilworth New Jersey 07033 (US)

(72) Inventor: Sherlock, Margaret Hagar
34 Parkway West
Bloomfield New Jersey 07033 (US)
Inventor: Smith, Sidney Randal
700 Spring Avenue
Ridgewood New Jersey 07400 (US)
Inventor: Siegel, Marvin Ira
507 Maple Hill Drive
Woodbridge New Jersey 07095 (US)

(74) Representative: von Kreisler, Alek, Dipl.-Chem. et al
Deichmannhaus am Hauptbahnhof
D-5000 Köln 1 (DE)

EP 0 196 024 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel tricyclic compounds which possess anti-allergic, antiinflammatory, cytoprotective activity and an immunosuppressing effect.

1,8-Naphthyridines useful for treating allergic reactions in mammals are known from EP—A—0 092 786 and especially from EP—A 0 127 135. In particular, EP—A 0 127 135 describes *inter alia* 1,8-naphthyridines having a fused [3,2-c] furan or pyran ring, which may be substituted with one or more alkyl groups having 1 to 6 carbon atoms or include a double bond.

The compounds of the present invention are compounds of the formula

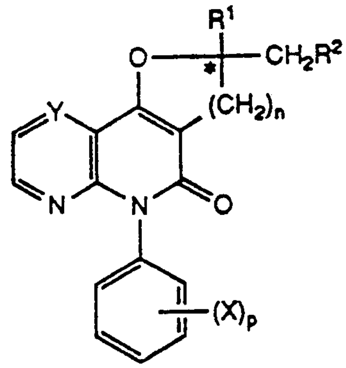

I

wherein n is 1;

$R^1$ and $R^2$ may be combined to form a bond, or $R^1$ is hydrogen and $R^2$ is OR, halogen or $NR^3R^4$;

R is hydrogen, $R^6$—C(O)—, or $R^7R^8NC(O)$—;

$R^3$ and $R^4$ are independently hydrogen or alkyl having from 1 to 6 carbon atoms, or $R^3$ and $R^4$ may be combined with the nitrogen to which they are attached to form a pyrrolidino, piperidino, morpholino or piperazino ring;

$R^6$ is alkyl having from 1 to 9 carbon atoms, alkenyl having from 2 to 7 carbon atoms, alkynyl having from 2 to 7 carbon atoms, phenyl, substituted phenyl (wherein the substituents are as defined for X) or benzyl;

$R^7$ and $R^8$ are independently hydrogen, alkyl having from 1 to 6 carbon atoms, or hydroxyalkyl having from 1 to 6 carbon atoms;

X is hydrogen, hydroxy, alkyl having from 1 to 6 carbon atoms, alkoxy having from 1 to 6 carbon atoms, nitro, halogen, trifluoromethyl or alkyl-$S(O)_m$ having from 1 to 6 carbon atoms, and wherein m is zero, 1 or 2;

p is 1, 2 or 3; and

Y is CH or N;

and the acid addition salts and/or solvates thereof.

Compounds of formula I wherein $R^1$ is hydrogen have at least one asymmetric carbon atom, i.e., the carbon indicated with an asterisk(*) in formula I. The compounds accordingly exist in enantiomeric forms or in racemic mixtures thereof, and all such isomers and racemic mixtures are within the scope of this invention. Separation of the isomers may be accomplished by methods well known to those skilled in the art.

The compounds of formula I can exist in unsolvated as well as solvated forms, including hydrated forms. In general, the solvated forms, with pharmaceutically acceptable solvents such as water and ethanol are equivalent to the unsolvated forms for purposes of the invention.

As used herein, the term "alkyl" refers to straight or branched chain groups, e.g. methyl, ethyl, propyl, isopropyl, butyl, isobutyl and hexyl. Examples of "alkoxy" groups are methoxy, ethoxy, isopropoxy, butoxy and hexoxy. "Halogen" refers to fluorine, chlorine, bromine and iodine.

The term "acid addition salts" as used herein refers to salts formed with pharmaceutically acceptable acids such as, for example, hydrochloric, hydrobromic, methane sulfonic and sulfuric acids.

A group of preferred compounds is that wherein Y is CH.

A further group of preferred compounds is that wherein $R^1$ is hydrogen and $R^2$ is OR wherein R is hydrogen or $R^6C(O)$—, preferably R being hydrogen or $R^6C(O)$—, wherein $R^6$ is alkyl.

A further group of preferred compounds is that wherein $R^1$ is hydrogen and $R^2$ is $NR^3R^4$, preferably being 1-piperidinyl or 1-pyrrolidinyl.

A further group of preferred compounds is that wherein $R^1$ is hydrogen and $R^2$ is halogen, preferably bromo or iodo.

Still another group of preferred compounds is that wherein p is 1 and X is hydrogen, halogen or alkoxy, preferably hydrogen, chloro or methoxy, preferably the substituent being in meta-position.

Also contemplated as part of this invention are pharmaceutical compositions which comprises a compound of formula I in combination with a pharmaceutically acceptable carrier.

The compounds of this invention are useful for treating allergic reactions and inflammation.

It is also contemplated that compounds of the invention are useful in the treatment of peptic ulcers.

The compounds of this invention are useful for suppressing the immune response in mammals.

The compounds of this invention can be prepared according to known methods. In the following equations suitable methods are described. Reactive groups not involved in the described reaction are protected according to standard methods before the reaction and are subsequently deprotected to yield the desired product. The starting compounds used in the described processes can be prepared according to known methods.

In the formulae of the followiong description of the processes, unless stated otherwise,

n is 1;

$R^1$ and $R^2$ may be combined to form a bond, or $R^1$ is hydrogen and $R^2$ is OR, halogen or $NR^3R^4$;

R is hydrogen, $R^6$—C(O)—, or $R^7R^8$NC(O)—;

$R^3$ and $R^4$ are independently hydrogen or alkyl having from 1 to 6 carbon atoms, or $R^6$ and $R^4$ may be combined with the nitrogen to which they are attached to form a pyrrolidino, piperidino, morpholino or piperazino ring;

$R^6$ is alkyl having from 1 to 9 carbon atoms, alkenyl having from 2 to 7 carbon atoms, alkynyl having from 2 to 7 carbon atoms, phenyl, substituted phenyl (wherein the substituents are as defined for X) or benzyl;

$R^7$ and $R^8$ are independently hydrogen, lower alkyl having from 1 to 6 carbon atoms, or hydroxyalkyl having from 1 to 6 carbon atoms;

X is hydrogen, hydroxy, alkyl having from 1 to 6 carbon atoms, alkoxy having from 1 to 6 carbon atoms, nitro, halogen, trifluoromethyl or alkyl-$S(O)_m$ having from 1 to 6 carbon atoms, and wherein m is zero, 1 or 2;

p is 1, 2 or 3; and

Y is CH or N; including suitable protecting groups.

The processes are particularly useful for the preparation of compounds of formula I wherein Y is CH.

(a) For the preparation of compounds of formula I wherein $R^1$ is hydrogen and $R^2$ is halogen, a compound of formula VIII

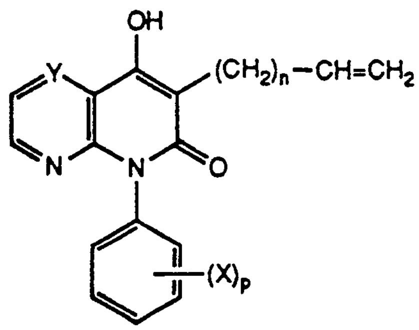

VIII

or an ester thereof is subjected to halogenation and cyclisation. This procedure is particularly useful for the preparation of compounds wherein Y is CH.

For example, for the preparation of compounds of formula I wherein $R^1$ is hydrogen and $R^2$ is bromo, a compound VIII or its 4-ester is subjected to bromination, which results in spontaneous cyclization of the intermediate dibromo compound. The bromination can be carried out at reduced temperatures (−10 to +32°C), by adding bromine to the solution of the compound VIII and letting the reaction mixture come to room temperature. Preferably equimolar amounts of compound VIII and bromine are used. After the isolation of the reaction product compounds I are isolated, for example, by column chromatography.

For the preparation of compounds of formula I wherein $R^1$ is hydrogen and $R^2$ is iodo, a compound of formula VIII or its 4-ester is reacted with N-iodosuccinimide in the presence of perchloric acid at reduced temperature. The compounds I are isolated by e.g., column chromatography.

b) A compound of formula IX

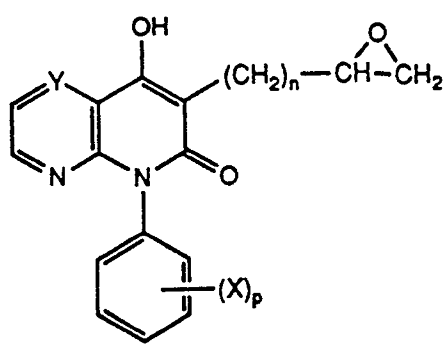

IX

or ester thereof is cyclized to form a compound of formula I wherein $R^1$ is hydrogen and $R^2$ is hydroxy.

The cyclization can be carried out by heating, if desired, a solution of compound of formula IX. The compound IX can be obtained by reacting the corresponding compound of formula VIII with peracetic acid in a solvent (e.g., ethylacetate or a halogenated hydrocarbon, e.g., methylene chloride) at 20 to 30°C, preferably around room temperature. Without isolating the compound IX the desired end product I can be obtained.

c) Compounds of formula I, wherein $R^1$ is hydrogen, $R^2$ is hydroxy can be prepared by hydrolysis of a compound of formula I, wherein $R^1$ is hydrogen and $R^2$ is halogen.

d) A compound of formula I wherein $R^1$ is hydrogen and $R^2$ is hydroxy can be esterified to form the corresponding respective compound of formula I. therein $R^1$ is hydrogen, $R^2$ is OR, wherein R is $R^6C(O)$— or $R^7R^8NC(O)$—, wherein $R^6$ is alkyl having from 1 to 9 carbon atoms, alkenyl having from 2 to 7 carbon atoms, phenyl, substituted phenyl (wherein the substituents are as defined for X) or benzyl and $R^7$ and $R^8$ are independently hydrogen, lower alkyl having from 1 to 6 carbon atoms, or hydroxyalkyl having from 1 to 6 carbon atoms. This process is carried out using acylating processes known in the art, such as reacting the hydroxy compound with the appropriate acid anhydride or acyl chloride in an inert solvent such as, for example, benzene.

e) A compound of formula I wherein $R^1$ is hydrogen and $R^2$ is halogen, preferably bromo, is reacted with an amine $HNR^3R^4$ (wherein $R^3$ and $R^4$ are independently hydrogen or alkyl having from 1 to 6 carbon atoms, or $R^3$ and $R^4$ may be combined with the nitrogen to which they are attached to form a pyrrolidino, piperidino, morpholino or piperazino ring) or with a reactive derivative of said amine to form the respective corresponding compound I wherein $R^1$ is hydrogen and $R^2$ is $NR^3R^4$. The reaction can be carried out at room temperature.

f) A compound of formula I wherein $R^1$ is hydrogen and $R^2$ is halogen, preferably iodo, is dehydrohalogenated to form the respective corresponding compound I wherein $R^1$ and $R^2$ together form a bond. The reaction can be carried out at room temperature in the presence of a base, preferably an organic base such as, for example, piperidine.

g) A compound of formula I, wherein $R^1$ is hydrogen, $R^2$ is OR, wherein R is $R^6$—C(O)—, or $R^7R^8NC(O)$—;

$R^6$ is alkyl having from 1 to 9 carbon atoms, alkenyl having from 2 to 7 carbon atoms, alkynyl having from 2 to 7 carbon atoms, phenyl, substituted phenyl (wherein the substituents are as defined for X) or benzyl;

$R^7$ and $R^8$ are independently hydrogen, lower alkyl having from 1 to 6 carbon atoms, or hydroxyalkyl having from 1 to 6 carbon atoms, can be hydrolyzed to yield the respective corresponding compound of formula I, wherein $R^1$ is hydrogen, $R^2$ is hydroxy. The hydrolysis can be carried out in an aqueous alkaline medium, if desired also containing an alcohol (e.g., methanol). Preferably the hydrolysis is carried out with NaOH (preferably 0.5 NaOH) under reflux of the aqueous alcohol-free medium.

h) A compound of formula I wherein $R^1$ is hydrogen and $R^2$ is hydroxy is dehydrated to form the corresponding respective compound of formula I wherein $R^1$ and $R^2$ together form a bond. The reaction can be carried out with dicyclohexylcarbodiimide (DCC) in the presence of a catalyst such as, for example, CuCl in an organic solvent such as an ether, preferably diethylether.

The above processes are followed by isolation of the individual compounds and/or formation of salts and/or solvates. The compounds in their enantiomeric form can be isolated from the racemic mixture according to known methods.

Compounds of formula VIII may be prepared by methods known in the art. See, for example, U.S. Patent 4,492,702. An example of such a procedure is provided in Preparation 1.

Representative of the compounds of this invention of formula I are the following:
3,5-dihydro-2-(hydroxymethyl)-5-phenyl-furo(3,2-c)-1,8-naphthyridin-4(2*H*)-one,
5-(3-chlorophenyl)-3,5-dihydro-2-(hydroxymethyl)-furo(3,2-c)-1,8-naphthyridin-4(2*H*)-one,
2-(acetyloxymethyl)-3,5-dihydro-5-phenyl-furo(3,2-c)-1,8-naphthyridin-4(2*H*)-one,
2-(acetyloxymethyl)-3,5-dihydro-5-(3-methoxyphenyl)-furo(3,2-c)-1,8-naphthyridin-4(2*H*)-one,
3,5-dihydro-2-(iodomethyl)-5-phenyl-furo(3,2-c)-1,8-naphthyridin-4(2*H*)-one,
3,5-dihydro-2-(iodomethyl)-5-(3-methoxyphenyl)-furo(3,2-c)-1,8-naphthyridin-4(2*H*)-one,
3,5-dihydro-5-phenyl-2-(1-pyrrolidinylmethyl)-furo(3,2-c)-1,8-naphthyridin-4(2*H*)-one,
3,5-dihydro-5-phenyl-2-(1-piperiidinylmethyl)-furo(3,2-c)-1,8-naphthyridin-4-(2*H*)-one,
2-(hydroxymethyl)-3,5-dihydro-5-(3-methoxyphenyl)-furo(3,2-c)-1,8-naphthyridin-4-(2*H*)-one,
(in the form of the racemic mixtures or the enantiomers) or
pharmaceutically acceptable salts or solvates thereof.

The compounds of this invention can be used to treat allergy caused diseases and their preferred use is for treating allergic chronic obstructive lung diseases. Chronic obstructive lung disease as used herein means disease conditions in which the passage of air through the lungs is obstructed or diminished such as is the case in asthma and bronchitis.

The anti-allergy method of this invention is identified by tests which measure a compound's inhibition of anaphylactic bronchospasm in sensitized rats having antigen induced bronchoconstriction. For example, the compound 3,5-dihydro-2-(hydroxymethyl)-5-phenyl-furo-[3,2-c]-1,8-naphthyridin-4(2*H*)-one was found to inhibit anaphylactic bronchospasm in such a test procedure when given at an oral dose of 2 mg/kg. Said compound was also found to inhibit allergen-induced histamine release from guinea pig sensitized tissue.

The compounds are effective non-adrenergic, non-anticholinergic antianaphylactic agents. When administered orally they are active at doses from about 0.1 to 10 mg/kg of body weight; when administered parenterally, e.g., intravenously, the compounds are active at dosages of from about 0.05 to 5 mg/kg body weight; when administered by inhalation (aerosol or nebulizer) the compounds are active at dosages of about 0.25 to 5 mg per puff, and one to four puffs may be taken every 4 hours.

The compounds of this invention are also useful for the treatment of inflammation. The anti-inflammatory use of the compounds of the present invention may be demonstrated by the Reversed Passive Arthus Reaction (RPAR) Rat Paw technique as set forth below. The potency of the compounds is determined using indomethacin as the standard. On the basis of the test results, an oral dosage range of about 5 milligrams per kilogram of body weight per day to about 50 milligrams per kilogram of body weight per day in divided doses taken at about 4 hour intervals is recommended.

The dosage to be administered and the route of administration depends upon the particular compound used, the age and general health of the patient and the severity of the inflammatory condition. Thus, the dose ultimately decided upon must be left to the judgment of a trained health-care practitioner.

RPAR Rat Paw Technique

Animals, Materials and Methods

Male Lewis inbred albino rats weighing 180—200 grams obtained from Charles River Breeding Laboratories are used in these experiments. The rats are housed 3 animals/cage and food and water are allowed *ad libitum.* The animals are numbered 1—3 in each cage and color marked for identification purposes.

Drug and Reagent Preparation

All reagents and drugs are prepared just prior to the study. Crystallized and lyophilized bovine serum albumin (BSA), obtained from Sigma Chemical Company, is solubilized without shaking in cold sterile pyrogen free saline (10 mg/ml). Lyophilized anti-bovine serum albumin (IGG fraction), obtained from Cappel Laboraatories, is suspended in sterile distilled water and diluted with cold pyrogen free saline (PFS) just prior to use. The final concentration of anti-bovine serum albumin is 0.5 mg/ml of PFS. Both BSA and anti-BSA solutions are iced during use. Drugs are suspended or solubilized in an aqueous solution of methyl cellulose (MC) with a homogenizer just prior to administration.

Drug Administration and Induction of Inflammation

Groups of animals (6/group) are dosed with drug in MC by gavage once daily for 3 days. The last dose is administered one hour prior to sensitization with BSA. Controls are given MC alone and a drug-standard is usually included in each assay for verification purposes. Drugs are prepared so as to provide a dose for a 200 gram animal which is equivalent to the mg/kg dose for the experiment. Thus each rat receives an oral dose in a volume of approximately 2.0 ml. One hour after the last dose the animals are lightly anesthetized with ether and "sensitized" by injection into the penile vein with 0.2 ml of PFS containing 1.0 mg of BSA. One hour later, the animals are "challenged" in the right rear paw with subplantar injections of 0.2 ml of PFS containing 0.1 mg of anti-BSA. Immediately after the subplantar injection, the right paw is dipped (up to the lateral maleolus) into the mercury well of a plethysmograph. The volume of mercury displaced is converted to weight and recorded. This value is considered to be the control reading for the animal. Paw volumes are also recorded with a plethysmograph during the development of the inflammation at 2 and 4 hours post-challenge.

Results

Results are expressed by the change in paw volume (A paw volume) from the control reading for each animal to that recorded 2 and 4 hours post-challenge. All drug treated groups are compared to the MC control for significant differences with an analysis of variance. Differences from control in drug-treated groups are expressed as percent change from control.

When administered parenterally, e.g. intravenously, the compounds are administered at a dosage range of about 0.01—10 mg/kg of body weight in single or multiple daily doses.

The compounds are also useful in the treatment of autoimmune and other immunological diseases including graft rejection in which T cell proliferation is a contributing factor to the pathogenesis of disease. The effectiveness of these compounds as immunosuppressing agents may be demonstrated by the following tests which involve the inhibition of T cell functions using these compounds.

Graft vs. Host Reaction (GVHR)

To induce a GVHR, C57 B1/6XA/J(B6AF1) male mice were injected intravenously with parental (C57B1/6J) spleen and lymph node cells. The compound 3,5-dihydro-2-hydroxymethyl-5-phenyl-furo(3,2-c)-1,8-naphthyridin-4(2*H*)-one (Compound A) was then administered orally for 10 days beginning on the day prior to the cell transfer. On the day following the last treatment, the animals were sacrificed, and their spleens were excised and weighed. The enlargement of the spleen of the host is a result of a GVHR. To some extent it is the host's own cells which infiltrate and enlarge the spleen although they do this because of the presence of graft cells reacting against the host. The amount of spleen enlargement, splenomegaly, is taken as a measure of the severity of the GVHR.

In carrying out the GVHR the animal in the experimental group is injected with parental cells, cells of the same species but of different genotype, which cause a weight increase of the spleen. The animal in the control group is injected with syngeneic cells, genetically identical cells which do not cause a weight increase of the spleen. The effectiveness of Compound A administered to the mice in the experimental group is measured by comparing the spleen weight of the untreated and treated GVH animal with that of the syngeneic control. In this test Compound A reduced spleen weight by 138% as compared to the untreated animals at a dose of 100 mg/kg.

Splenic Atrophy

The immunosuppressive activity of the compounds may also be shown by a decrease in spleen weight after dosing $BDF_1$ mice orally with the drug for seven (7) consecutive days. The mice are sacrificed on the eighth day. The percent decrease in spleen weight is measured for each dosage level. In this procedure Compound A provided a 25% and 48% spleen weight decrease at a dosage level of 25 mg/kg and 50 mg/kg, respectively, indicating an $ED_{50}$ of between 25 mg/kg and 50 mg/kg.

As mentioned above, the subject compounds also possess anti-allergy and anti-inflammatory activities. For example, Compound A has an $ED_{50}$ value of below about 2 mg/kg p.o. in tests measuring the inhibition of anaphylactic bronchospasm in sensitized guinea pigs having antigen-induced bronchoconstriction and an $ED_{50}$ value of below about 25 mg/kg p.o. in tests measuring the reverse passive Arthus reaction in the paw of rats (as described above). These results for Compound A indicate that an immunosuppressive effective dose for the compounds of formula I is about 5 times or more their anti-allergy effective doses ($ED_{50s}$).

The usual dosage range for the compounds of formula I in a 70 kg mammal is an oral dose of about .1 to 250 mg/kg, preferably .1 to 150 mg/kg, in 3 or 4 divided doses per day. Of course, the dose will be regulated according to the potency of compound employed, the immunological disease being treated, and the judgment of the attending clinician depending on factors such as the degree and the severity of the disease state and age and general condition of the patient being treated.

Example 6

3,5-Dihydro-5-(3-chlorophenyl)-2-(hydroxymethyl)-furo(3,2-c)-1,8-naphthyridin-4(2H)-one

In a manner similar to that described in Example 1, treat 2-(bromomethyl)-9-(3-chlorophenyl)-3,9-dihydro-furo(2,3-b)-1,8-naphthyridin-4(2H)-one to yield the title compound, m.p. 243—245°C.

The following Formulation Examples are intended to illustrate the present invention. In the examples the term "Compound A" refers to 3,5-dihydro-2-hydroxymethyl-5-phenyl-furo(3,2-c)-1,8-naphthyridin-4(2*H*)-one. It is contemplated, however, that this compound may be replaced by equally effective quantities of other compounds of formula I as defined above.

Formulation 1

Tablets

| No. | Ingredient | mg/tablet | mg/tablet |
|---|---|---|---|
| 1. | Compound A | 100 | 500 |
| 2. | Lactose USP | 122 | 113 |
| 3. | Corn Starch, Food Grade, as a 10% paste in Purified Water | 30 | 40 |
| 4. | Corn Starch, Food Grade | 45 | 40 |
| 5. | Magnesium Stearate | 3 | 7 |
| | Total | 300 | 700 |

Method of Manufacture

Mix Item Nos. 1 and 2 in a suitable mixer for 10—15 minutes. Granulate the mixture with Item No. 3. Mill the damp granules through a coarse screen if needed. Dry the damp granules. Screen the dried granules if needed and mix with the Item No. 4 and mix for 10—15 minutes. Add Item No. 5 and mix for 1—3 minutes. Compress the mixture to appropriate the size and weight on a suitable tablet machine.

Formulation 2

Capsules

| No. | Ingredient | mg/capsule | mg/capsule |
|---|---|---|---|
| 1. | Compound A | 100 | 500 |
| 2. | Lactose USP | 106 | 123 |
| 3. | Corn Starch, Food Grade | 40 | 70 |
| 4. | Magnesium Stearate NF | 4 | 7 |
| | Total | 250 | 700 |

Method of Manufacture

Mix Item Nos. 1, 2 and 3 in a suitable blender, for 10—15 minutes. Add Item No. 4 and mix for 1—3 minutes. Fill the mixture into suitable two-piece hard gelatin capsules on a suitable encapsulating machine.

Formulation 3

Parenteral

| Ingredient | mg/vial | mg/vial |
|---|---|---|
| Compound A Sterile Powder | 100 | 500 |

Formulation 4

Injectable

| No. | Ingredient | mg/vial | mg/vial |
|---|---|---|---|
| 1. | Compound A | 100 | 500 |
| 2. | Methyl para-hydroxybenzoate | 1.8 | 1.8 |
| 3. | Propyl para-hydroxybenzoate | 0.2 | 0.2 |
| 4. | Sodium Bisulfite | 3.2 | 3.2 |
| 5. | Disodium Edetate | 0.1 | 0.1 |
| 6. | Sodium Sulfate | 2.6 | 2.6 |
| 7. | Water for Injection g.s. ad | 1.0 ml | 1.0 ml |

Method for Manufacture

1. Dissolve the hydroxybenzoate compounds in a portion (85% of the final volume) of the water for injection at 65—70°C.
2. Cool to 25—35°C. Charge and dissolve the sodium bisulfite, disodium edetate and sodium sulfate.
3. Charge and dissolve drug.
4. Bring the solution to final volume by added water for injection.
5. Filter the solution through 0.22 membrane and fill into appropriate containers.
6. Finally sterilize the units by autoclaving.

**Claims**

1. A compound of the formula

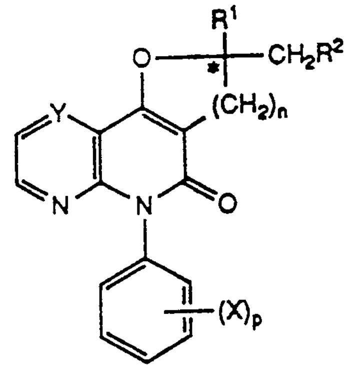

I

wherein n is 1;

$R^1$ and $R^2$ may be combined to form a bond, or $R^1$ is hydrogen and $R^2$ is OR, halogen or $NR^3R^4$;

R is hydrogen, $R^6$—C(O)—, or $R^7R^8NC(O)$—;

$R^3$ and $R^4$ are independently hydrogen or alkyl having from 1 to 6 carbon atoms, or $R^3$ and $R^4$ may be combined with the nitrogen to which they are attached to form a pyrrolidino, piperidino, morpholino or piperazino ring;

$R^6$ is alkyl having from 1 to 9 carbon atoms, alkenyl having from 2 to 7 carbon atoms, alkynyl having from 2 to 7 carbon atoms, phenyl, substituted phenyl (wherein the substituents are as defined for X) or benzyl;

$R^7$ and $R^8$ are independently hydrogen, alkyl having from 1 to 6 carbon atoms, or hydroxyalkyl having from 1 to 6 carbon atoms;

X is hydrogen, hydroxy, alkyl having from 1 to 6 carbon atoms, alkoxy having from 1 to 6 carbon atoms, nitro, halogen, trifluoromethyl or alkyl-$S(O)_m$ having from 1 to 6 carbon atoms, and wherein m is zero, 1 or 2;

p is 1, 2 or 3; and

Y is CH or N; and the acid addition salts and/or solvates thereof.

2. A compound according to claim 1, wherein Y is CH.

3. A compound according to claim 2, wherein $R^1$ is hydrogen and $R^2$ is OR, wherein R is hydrogen or $R^6C(O)$— wherein $R^6$ is alkyl, or $R^2$ is $NR^3R^4$.

4. A compound according to claim 2, wherein $R^1$ is hydrogen and $R^2$ is OR, wherein R is hydrogen or $R^6C(O)$ wherein $R^6$ is alkyl, or $R^2$ is 1-piperidinyl or 1-pyrrolidinyl.

5. A compound according to claim 2, wherein $R^1$ is hydrogen and $R^2$ is halogen.

6. A compound according to claim 2, wherein $R^2$ is bromo or iodo.

7. A compound according to any of claims 1 to 6 wherein p is 1 and X is hydrogen, halogen or alkoxy.

8. A compound according to claim 7 wherein p is 1 and X is hydrogen, chloro or methoxy.

9. A compound according to claim 1 which is

3,5-dihydro-2-(hydroxymethyl)-5-phenylfuro[3,2-c]-1,8-naphthyridin-4(2*H*)-one,

5-(3-chlorophenyl)-3,5-dihydro-2-(hydroxymethyl)-furo[3,2-c]-1,8-naphthyridin-4(2*H*)-one,

2-(acetyloxymethyl)-3,5-dihydro-5-phenylfuro[3,2-c]-1,8-naphthyridin-4(2*H*)-one,

2-(acetyloxymethyl)-3,5-dihydro-5-(3-methoxyphenyl)-furo[3,2-c]-1,8-naphthyridin-4(2*H*)-one,

3,5-dihydro-2-(iodomethyl)-5-phenylfuro[3,2-c]-1,8-naphthyridin-4(2*H*)-one,

3,5-dihydro-2-(iodomethyl)-5-(3-methoxyphenylfuro[3,2-c]-1,8-naphthyridin-4(2*H*)-one,

3,5-dihydro-5-phenyl-2-(1-pyrrolidinylmethyl)-furo[3,2-c]-1,8-naphthyridin-4(2*H*)-one,

3,5-dihydro-5-phenyl-2-(1-piperidinylmethyl)-furo[3,2-c]-1,8-naphthyridin-4(2*H*)-one,

2-(hydroxymethyl)-3,5-dihydro-5-(3-methoxyphenyl)-furo[3,2-c]-1,8-naphthyridin-4(2*H*)-one,

(in the form of the racemic mixtures or the enantiomers) or pharmaceutically acceptable salts thereof.

10. Process for the preparation of a compound of the formula I or its acid addition salt and/or solvates as defined in claim 1, characterized in that

a) a compound of formula VIII

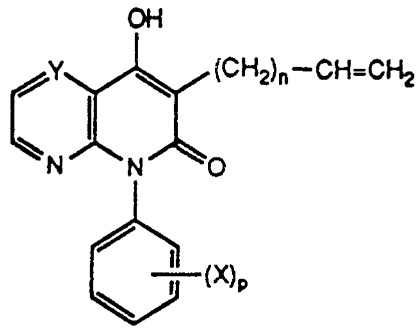

VIII

or an ester thereof is subjected to halogenation and cyclization to form a compound of formula I wherein $R^1$ is hydrogen and $R^2$ is halogen; or

b) a compound of formula IX

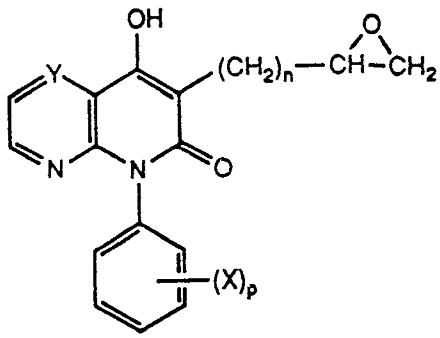

IX

is cyclized to form a compound of formula I wherein $R^1$ is hydrogen and $R^2$ is hydroxy; or

c) a compound of formula I

wherein $R^1$ is hydrogen and $R^2$ is halogen is hydrolyzed to give a compound of formula I wherein $R^1$ is hydrogen and $R^2$ is hydroxy; or

d) a compound of formula I wherein $R^1$ is hydrogen and $R^2$ is hydroxy is esterified to yield the corresponding compound of formula I wherein $R^1$ is hydrogen and $R^2$ is OR wherein R is $R^6C(O)$— or $R^7R^8NC(O)$— and $R^7$ and $R^8$ are independently hydrogen, alkyl having from 1 to 6 carbon atoms, or hydroxyalkyl having from 1 to 6 carbon atoms; or

e) a compound of formula I wherein $R^1$ is hydrogen and $R^2$ is halogen is reacted with an amine $HNR^3R^4$ (wherein $R^3$ and $R^4$ are independently hydrogen or alkyl having from 1 to 6 carbon atoms, or $R^3$ and $R^4$ may be combined with the nitrogen to which they are attached to form a pyrrolidino, piperidino, morpholino or piperazino ring) or with a reactive derivative of said amine to form the corresponding compound of the formula I wherein $R^1$ is hydrogen and $R^2$ is $NR^3R^4$; or

f) a compound of formula I wherein $R^1$ is hydrogen and $R^2$ is halo is dehydrohalogenated to form the corresponding compound of the formula I wherein $R^1$ and $R^2$ together form a bond; or

g) a compound of formula I, wherein $R^1$ is hydrogen and $R^2$ is OR, wherein

R is $R^6$—C(O)— or $R^7R^8NC(O)$—;

$R^6$ is alkyl having from 1 to 9 carbon atoms, alkenyl having from 2 to 7 carbon atoms, alkynyl having from 2 to 7 carbon atoms, phenyl, substituted phenyl (wherein the substituents are as defined for X) or benzyl;

$R^7$ and $R^8$ are independently hydrogen, alkyl having from 1 to 6 carbon atoms, or hydroxyalkyl having from 1 to 6 carbon atoms, is hydrolyzed to yield the corresponding compound of formula I wherein $R^1$ is hydrogen and $R^2$ is hydroxy; or

h) a compound of formula I wherein $R^1$ is hydrogen and $R^2$ is hydroxy is dehydrated to yield the corresponding compound of the formula I wherein $R^1$ and $R^2$ together form a bond;

whereby each of the processes is followed, if desired, by isolation of the individual compound and/or formation of the salt and/or solvate of the compound(s).

11. A process as claimed in claim 10, process e), wherein $R^2$ is bromo.

12. A pharmaceutical composition which comprises a compound of any of claims 1 to 9 in combination with a pharmaceutically acceptable carrier.

13. A compound of any one of claims 1 to 9 for use in eliciting an anti-allergic and/or anti-inflammatory and/or cytoprotective and/or immunosuppressing effect.

**Patentansprüche**

1. Verbindung der Formel

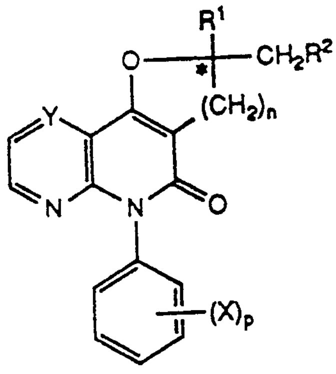

I

in der

n 1 ist;

$R^1$ und $R^2$ kombiniert werden können, wodurch eine Bindung gebildet wird, oder $R^1$ Wasserstoff ist und $R^2$ OR, Halogen oder $NR^3$ $R^4$ ist;

R Wasserstoff, $R^6$—C(O)— oder $R^7R^8NC(O)$— ist;

$R^3$ und $R^4$ unabhängig voneinander Wasserstoff oder Alkyl mit 1 bis 6 Kohlenstoff-Atomen sind oder $R^3$ und $R^4$ mit dem Stickstof, an den sie gebunden sind, kombiniert werden können und einen Pyrrolidin-, Piperidin-, Morpholin- oder Piperazin-Ring bilden;

$R^6$ Alkyl mit 1 bis 9 Kohlenstoff-Atomen, Alkenyl mit 2 bis 7 Kohlenstoff-Atomen, Alkinyl mit 2 bis 7 Kohlenstoff-Atomen, Phenyl, substituiertes Phenyl (worin die Substituenten den Definition für X entsprechen) oder Benzyl ist,

$R^7$ und $R^8$ unabhängig voneinander Wasserstoff, Alkyl mit 1 bis 6 Kohlenstoff-Atomen oder Hydroxyalkyl mit 1 bis 6 Kohlenstoff-Atomen sind;

X Wasserstoff, Hydroxy, Alkyl mit 1 bis Kohlenstoff-Atomen, Alkoxy mit 1 bis 6 Kohlenstoff-Atomen, Nitro, Halogen, Trifluoromethyl oder Alkyl-$S(O)_m$ mit 1 bis 6 Kohlenstoff-Atomen ist, worin m Null, 1 oder 2 ist;

p 1, 2 oder 3 ist; und

Y CH oder N ist;

und die Säureadditionssalze und/oder Solvate derselben.

2. Verbindung nach Anspruch 1, worin Y CH ist.

3. Verbindung nach Anspruch 2, worin $R^1$ Wasserstoff ist und $R^2$ OR ist, worin R Wasserstoff oder $R^6C(O)$— ist, worin $R^6$ Alkyl ist, oder $R^2$ $NR^3R^4$ ist.

4. Verbindung nach Anspruch 2, worin $R^1$ Wasserstoff ist und $R^2$ OR ist, worin R Wasserstoff oder $R^6C(O)$— ist, worin $R^6$ Alkyl ist, oder $R^2$ 1-Piperidinyl oder 1-Pyrrolidinyl ist.

5. Verbindung nach Anspruch 2, worin $R^1$ Wasserstoff ist und $R^2$ Halogen ist.

6. Verbindung nach Anspruch 2, worin $R^2$ Bromo oder Iodo ist.

7. Verbindung nach irgendeinem der Ansprüche 1 bis 6, worin p 1 ist und X Wasserstoff, Halogen oder Alkoxy ist.

8. Verbindung nach Anspruch 7, worin p 1 ist und X Wasserstoff, Chloro oder Methoxy ist.

9. Verbindung nach Anspruch 1, die

3,5-Dihydro-2-(hydroxymethyl)-5-phenylfuro[3,2-c]-1,8-naphthyridin-4(2*H*)-on,
5-(3-Chlorophenyl)-3,5-dihydro-2-(hydroxymethyl)furo[3,2-c]-1,8-naphthyridin-4(2*H*)-on,
2-(Acetyloxymethyl)-3,5-dihydro-5-phenylfuro[3,2-c]-1,8-naphthyridin-4(2*H*)-on,
2-(Acetyloxymethyl)-3,5-dihydro-5-(3-methoxyphenyl)-furo[3,2-c]-1,8-naphthyridin-4(2*H*)-on,
3,5-Dihdro-2-(iodomethyl)-5-phenylfuro[3,2-c]-1,8-naphthyridin-4(2*H*)-on,
3,5-Dihydro-2-(iodomethyl)-5-(3-methoxyphenyl)furo[3,2-c]-1,8-naphthyridin-4(2*H*)-on,
3,5-Dihydro-5-phenyl-2-(1-pyrrolidinylmethyl)furo[3,2-c]-1,8-naphthyridin-4(2*H*)-on,
3,5-Dihydro-5-phenyl-2-(1-piperidinylmethyl)furo[3,2-c]-1,8-naphthyridin-4(2*H*)-on,
2-(Hydroxymethyl)-3,5-dihydro-5-(3-methoxyphenyl)furo[3,2-c]-1,8-naphthyridin-4(2*H*)-on

(in Form der racemischen Gemische oder der Enantiomeren) ist, oder deren pharmazeutisch annehmbare Salze.

10. Verfahren zur Herstellung einer Verbindung der Formel I oder eines Säureadditionssalzes derselben und/oder von Solvaten, wie sie in Anspruch 1 definiert sind, dadurch gekennzeichnet, daß

a) eine Verbindung der Formel VIII

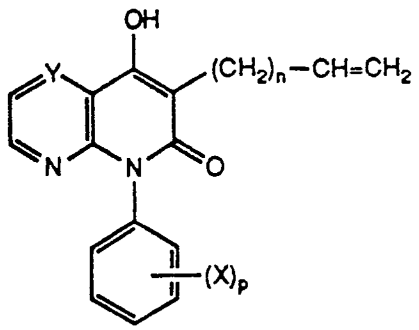

VIII

oder ein Ester derselben der Halogenierung und Cyclisierung unterworfen wird, um eine Verbindung der Formel I zu bilden, in der $R^1$ Wasserstoff ist und $R^2$ Halogen ist; oder

b) eine Verbindung der Formel IX

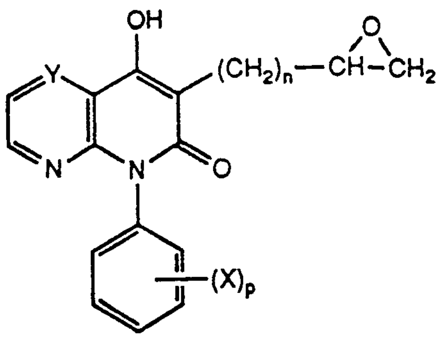

IX

zur Verbindung der Formel I cyclisiert wird, in der $R^1$ Wasserstoff ist und $R^2$ Hydroxy ist; oder

c) eine Verbindung der Formel I, in der $R^1$ Wasserstoff ist und $R^2$ Halogen ist, hydrolysiert wird, um eine Verbindung der Formel I zu ergeben, in der $R^1$ Wasserstoff ist und $R^2$ Hydroxy ist; oder

d) eine Verbindung der Formel I, in der $R^1$ Wasserstoff ist und $R^2$ Hydroxy ist, verestert wird, um eine Verbindung der Formel I zu ergeben, in der $R^1$ Wasserstoff ist und $R^2$ OR ist, worin R $R^6$—C(O)— oder $R^7R^8NC(O)$— ist und $R^7$ und $R^8$ unabhängig voneinander Wasserstoff, Alkyl mit 1 bis 6 Kohlenstoff-Atomen oder Hydroxyalkyl mit 1 bis 6 Kohlenstoff-Atomen sind; oder

e) eine Verbindung der Formel I, in der $R^1$ Wasserstoff ist und $R^2$ Halogen ist, mit einem Amin $HNR^3R^4$ (worin $R^3$ und $R^4$ unabhängig voneinander Wasserstoff oder Alkyl mit 1 bis 6 Kohlenstoff-Atomen sind oder $R^3$ und $R^4$ mit dem Stickstoff, an den sie gebunden sind, kombiniert werden können und einen Pyrrolidin-, Piperidin-, Morpholin- oder Piperazin-Ring bilden) oder mit einem reaktionsfähigen Derivat des Amins umgesetzt werden, um die entsprechende Verbindung der Formel I zu bilden, in der $R^1$ Wasserstoff ist und $R^2$ $NR^3R^4$ ist; oder

f) eine Verbindung der Formel I, in der $R^1$ Wasserstoff ist und $R^2$ Halogen ist, enthydrohalogeniert wird, um die entsprechende Verbindung der Formel I zu bilden, in der $R^1$ und $R^2$ gemeinsam eine Bindung bilden; oder

g) eine Verbindung der Formel I, in der $R^1$ Wasserstoff ist und $R^2$ OR ist, worin R $R^6$—C(O)— oder $R^7R^8NC(O)$— ist,

$R^6$ Alkyl mit 1 bis 9 Kohlenstoff-Atomen, Alkenyl mit 2 bis 7 Kohlenstoff-Atomen, Alkinyl mit 2 bis 7 Kohlenstoff-Atomen, Phenyl, substituiertes Phenyl (worin die Substituenten die für X angegebenen Bedeutungen haben) oder Benzyl ist,

$R^7$ und $R^8$ unabhängig voneinander Wasserstoff, Alkyl mit 1 bis 6 Kohlenstoff-Atomen oder Hydroxyalkyl mit 1 bis 6 Kohlenstoff-Atomen sind, hydrolysiert wird, um eine Verbindung der Formel I zu ergeben, in der $R^1$ Wasserstoff ist und $R^2$ Hydroxy ist; oder

h) eine Verbindung der Formel I, in der $R^1$ Wasserstoff ist und $R^2$ Hydroxy ist, dehyradatisiert wird, um die entsprechende Verbindung der Formel I zu bilden, in der $R^1$ und $R^2$ gemeinsam eine Bindung bilden; wobei im Anschluß an jedes Verfahren gewünschtenfalls die Isolierung der einzelnen Verbindung und/oder die Bildung des Salzes und/oder Solvats der Verbindung(en) erfolgen.

11. Verfahren nach Anspruch 10, Verfahren e), worin $R^2$ Bromo ist.

12. Pharmazeutische Zusammensetzung, umfassend eine Verbindung nach irgendeinem der Ansprüche 1 bis 9 in Kombination mit einem pharmazeutisch annehmbaren Träger.

13. Verbindung nach irgendeinem der Ansprüche 1 bis 9 zur Verwendung bei der Auslösung eines antiallergischen und/oder antiinflammatorischen und/oder cytoprotektiven und/oder immunosuppressiven Effekts.

**Revendications**

1. Composé de formule

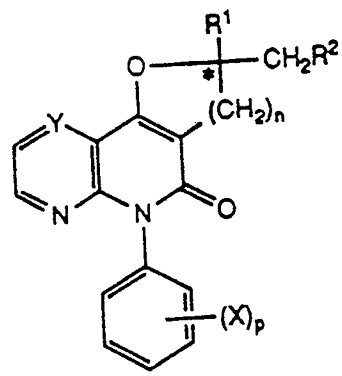

I

dans laquelle n est 1;

$R^1$ et $R^2$ peuvent être combinés pour former une liaison, ou bien $R^1$ est l'hydrogène et $R^2$ est OR, halogène ou $NR^3R^4$;

R est hydrogène, $R^6$—C(O)—, ou $R^7R^8NC(O)$—;

$R^3$ et $R^4$ sont indépendamment hydrogène ou alkyle possédant de 1 à 6 atomes de carbone, ou $R^3$ et $R^4$ peuvent être combinés avec l'azote auquel ils sont attachés pour former un cycle pyrrolidino, pipéridino, morpholino ou pipérazino;

$R^6$ est un groupe alkyle possédant de 1 à 9 atomes de carbone, alcényle ayant de 2 à 7 atomes de carbone, alcynyle ayant de 2 à 7 atomes de carbone, phényle, phényle substitué (dans lequel les substituants sont comme définis pour X) ou benzyle;

$R^7$ et $R^8$ sont indépendamment hydrogène, alkyle ayant de 1 à 6 atomes de carbone, ou hydroxyalkyle ayant de 1 à 6 atomes de carbone;

X est hydrogène, hydroxy, alkyle ayant de 1 à 6 atomes de carbone, alcoxy ayant de 1 à 6 atomes de carbone, nitro, halogène, trifluorométhyle, ou alkyle-$S(O)_m$ ayant de 1 à 6 atomes de carbone, et dans lequel

m est zéro, 1 ou 2;

p est 1, 2 ou 3; et

Y est CH ou N; ainsi que les sels d'addition d'acide et/ou les solvates de ce composé.

2. Composé suivant la revendication 1, caractérisé en ce que Y est CH.

3. Composé suivant la revendication 2, caractérisé en ce que $R^1$ est l'hydrogène et $R^2$ est OR, R étant l'hydrogène ou $R^6C(O)$— dans lequel $R^6$ est alkyle, ou $R^2$ est $NR^3R^4$.

4. Composé suivant la revendication 2, caractérisé en ce que $R^1$ est l'hydrogène et $R^2$ est OR, dans lequel R est l'hydrogène ou $R^6C(O)$ dans lequel $R^6$ est alkyle, ou bien $R^2$ est 1-pipéridinyle ou 1-pyrrolidinyle.

5. Composé suivant la revendication 2, caractérisé en ce que $R^1$ est l'hydrogène et $R^2$ est un halogène.

6. Composé suivant la revendication 2, caractérisé en ce que $R^2$ est bromo ou iodo.

7. Composé suivant l'une quelconque des revendications 1 à 6, caractérisé en ce que p est 1 et X est l'hydrogène, un halogène ou alcoxy.

8. Composé suivant la revendication 7, caractérisé en ce que p est 1 et X est l'hydrogène, chloro ou méthoxy.

9. Composé suivant la revendication 1, caractérisé en ce qu'il est constitué par:

la 3,5-dihydro-2-(hydroxyméthyl)-5-phényl-furo-[3,2-c]-1,8-naphtyridin-4(2*H*)-one,

la 5-(3-chlorophényl)-3,5-dihydro-2-(hydroxyméthyl)-furo-[3,2-c]-1,8-naphtyridin-4(2*H*)-one,

la 2-(acétyloxyméthyl)-3,5-dihydro-5-phényl-furo-[3,2-c]-1,8-naphtyridin-4(2*H*)-one,

la 2-(acétyloxyméthyl)-3,5-dihydro-5-(3-méthoxyphenyl)-furo-[3,2-c]-1,8-naphtyridin-4(2*H*)-one,

la 3,5-dihydro-2-(iodométhyl)-5-phényl-furo-[3,2-c]-1,8-naphtyridin-4(2*H*)-one,

la 3,5-dihydro-2-(iodométhyl)-5-(3-méthoxyphényl)-furo-[3,2-c]-1,8-naphtyridin-4(2*H*)-one,

la 3,5-dihydro-5-phényl-2-(1-pyrrolidinylméthyl)-furo-[3,2-c]-1,8-naphtyridin-4(2*H*)-one,

la 3,5-dihydro-5-phényl-2-(1-pipéridinylméthyl)-furo-[3,2-c]-1,8-naphtyridin-4(2*H*)-one,

la 2-(hydroxyméthyl)-3,5-dihydro-5-(3-méthoxyphényl)-furo[3,2-c]-1,8-naphtyridin-4(2*H*)-one, sous la forme de mélanges racémiques ou d'énantiomères ou les pharmaceutiquement acceptables de ces composés.

10. Procédé pour la préparation d'un composé de formule I ou de son del d'addition d'acide et/ou de ses solvates comme défini dans la revendication 1, caractérisé en ce que:

a) un composé de formule VIII

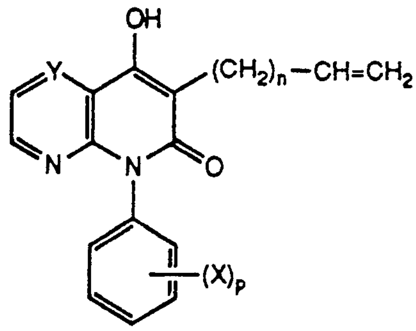

VIII

ou un ester de ce composé est soumis à une halogénation et une cyclisation pour former un composé de formule I dans laquelle $R^1$ est l'hydrogène et $R^2$ est un halogène; ou bien

b) un composé de formule IX

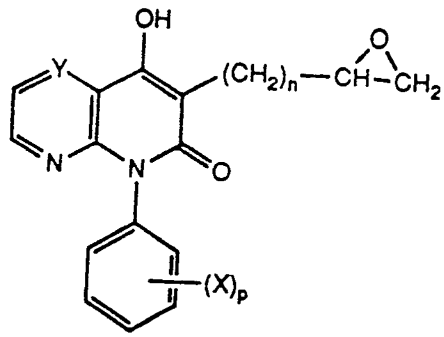

IX

est cyclisé pour former un composé de formule I dans laquelle $R^1$ est l'hydrogène et $R^2$ est hydroxy; ou bien

c) un composé de formule I dans laquelle $R^1$ est l'hydrogène et $R^2$ est un halogène, est hydrolysé pour donner un composé de formule I dans laquelle $R^1$ est l'hydrogène et $R^2$ est hydroxy; ou bien

d) un composé de formule I dans laquelle $R^1$ est l'hydrogène et $R^2$ est hydroxy est estérifié pour donner le composé correspondant de formule I dans laquelle $R^1$ est l'hydrogène et $R^2$ est OR dans lequel R est $R^6C(O)$— ou $R^7R^8NC(O)$— et $R^7$ et $R^8$ sont indépendamment l'hydrogène, un groupe alkyle ayant de 1 à 6 atomes de carbone, ou un groupe hydroxyalkyle ayant de 1 à 6 atomes de carbone; ou bien

e) on fait réagir un composé de formule I dans laquelle $R^1$ est l'hydrogène et $R^2$ est un hahlogène avec une amine $HNR^3R^4$ (dans laquelle $R^3$ et $R^4$ sont indépendamment hydrogène ou alkyle ayant de 1 à 6 atomes de carbone, ou $R^3$ et $R^4$ peuvent être combinés avec l'azote auquel ils sont attachés pour former un cycle pyrrolidino, pipéridino, morpholino ou pipérazino) ou bien avec un dérivé réactif de ladite amine pour former le composé correspondant de formule I dans laquelle $R^1$ est l'hydrogène et $R^2$ est $NR^3R^4$; ou bien

f) un composé de formule I dans laquelle $R^1$ est l'hydrogène et $R^2$ est halo est déshydrohalogéné pour former le composé correspondant de formule I dans laquelle $R^1$ et $R^2$ ensemble forment une liaison ; ou bien

g) un composé de formule I, dans laquelle $R^1$ est l'hydrogène et $R^2$ est OR, dans lequel

R est $R^6$—C(O)— ou $R^7R^8NC(O)$—;

$R^6$ est un groupe alkyle ayant de 1 à 9 atomes de carbone, alcényle ayant de 2 à 7 atomes de carbone, alcynyle ayant de 2 à 7 atomes de carbone, phényle, phényle substitué (où les substituants sont comme définis pour X) ou benzyle;

$R^7$ et $R^8$ sont indépendamment l'hydrogène, un groupe alkyle ayant de 1 à 6 atomes de carbone, ou hydroxyalkyle ayant de 1 à 6 atomes de carbone, est hydrolysé pour donner le composé correspondant de formule I où $R^1$ est l'hydrogène et $R^2$ est hydroxy; ou bien

h) un composé de formule I dans laquelle $R^1$ est l'hydrogène et $R^2$ est hydroxy est déshydraté pour donner le composé correspondant de formule I dans laquelle $R^1$ et $R^2$ ensemble forment une liaison;

chacun de ces procédés étant suivi, si on le désire, par une isolation du composé individuels et/ou la formation du sel et/ou du solvate du ou des composés.

11. Procédé suivant la revendication 10, caractérisé en ce que dans le procédé e) précité, $R^2$ est bromo.

12. Composition pharmaceutique comprenant un composé suivant l'une quelconque des revendications 1 à 9 en combinaison avec un véhicule pharmaceutiquement acceptable.

13. Composé suivant l'une quelconque des revendications 1 à 9, destiné à être utilisé pour provoquer un effect antiallergique et/ou anti-inflammatoire et/ou cytoprotecteur et/ou immuno-suppresseur.